# EUROPEAN PATENT APPLICATION

(11) **EP 3 332 769 A1**
(43) Date of publication of application: **13.06.2018**
(21) Application number: 17205587.3
(22) Date of filing: 06.12.2017
(51) Int. Cl.: A61K 9/20, A61K 31/519

(54) **SOLID ORAL PHARMACEUTICAL COMPOSITIONS OF TICAGRELOR**

(30) Priority: 07.12.2016 TR 201617983
(71) Applicant: Sanovel Ilac Sanayi ve Ticaret A.S., 34460 Istanbul (TR)
(72) Inventor: TÜRKYILMAZ, Ali, 34460 Istanbul (TR); YILDIRIM, Ediz, 34460 Istanbul (TR); KARABULUT, Irem, 34460 Istanbul (TR); ATMACA, Ibrahim, 34460 Istanbul (TR)
(74) Representative: Sevinç, Erkan

(57) **Abstract**

The present invention relates to a solid oral pharmaceutical composition, comprising ticagrelor or a pharmaceutically acceptable salt thereof with at least one hydrophilic polymer.

## Description

### Field of Invention

The present invention relates to a solid oral pharmaceutical composition, comprising ticagrelor or a pharmaceutically acceptable salt thereof with at least one hydrophilic polymer.

### Background of Invention

Ticagrelor (trade name BRILINTA®) is a platelet aggregation inhibitor produced by AstraZeneca. An antiplatelet drug is a member of a class of pharmaceuticals that decrease platelet aggregation and inhibit thrombus formation. They are effective in the arterial circulation, where anticoagulants have little effect. They are widely used in primary and secondary prevention of thrombotic cerebrovascular or cardiovascular disease.

Antiplatelet drug decreases the ability of blood clot to form by interfering with platelet activation process in primary haemostasis. Antiplatelet drugs can reversibly or irreversibly inhibit the process involved in platelet activation resulting in decreased tendency of platelets to adhere to one another and to damaged blood vessels endothelium.

Ticagrelor is a cyclo-pentyltriazolo-pyrimidine that reversibly inhibits the P2Y₁₂ receptor which is a protein found mainly but not exclusively on the surface of blood platelets and which is an important regulator in blood clotting.

The chemical name of ticagrelor is (1 S,2S,3R,5S)-3-[7-[(1 R,2S)-2-(3,4-Difluorophenyl) cyclopropylamino]-5-(propylthio)-3H-[1,2,3]triazolo[4,5-d]pyrimidin-3-yl] -5-(2-hydroxyethoxy) cyclopentane-1,2-diol and its chemical structure is shown in the Formula 1.

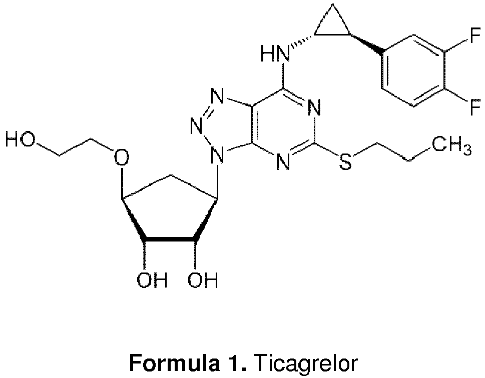

Ticagrelor (BRILINTA®) is indicated to reduce the rate of cardiovascular death, myocardial infarction, and stroke in patients with acute coronary syndrome (ACS) or a history of myocardial infarction (MI). For at least the first 12 months following ACS, it is superior to clopidogrel. It also reduces the rate of stent thrombosis in patients who have been stented for treatment of ACS.

In the state of the art, the patent application no. EP2056832A1 discloses an oral pharmaceutical composition comprising ticagrelor and other excipients. The composition is presented as enhancing the bioavailability through increasing the release of the active agent due to the formulation comprising at least two fillers and one single binder. Moreover, one of these fillers is dibasic calcium phosphate dehydrate which is commonly known by its abrasive properties. Considering these undesirable properties, the use of dibasic calcium phosphate dehydrate as a tablet excipient shall increase the required amount of lubricants (Handbook of Pharmaceutical Excipients, sixth edition, page 97). However, the ratio of the lubricant given in the application, which is preferably magnesium stearate, is in the range of 0.5-1% by weight. This low ratio is likely to cause stability problems in the presence of dibasic calcium phosphate dehydrate. On the other hand, the increase of the magnesium stearate amount shall reduce the dissolution rate and the promised enhanced bioavailability accordingly (Handbook of Pharmaceutical Excipients, sixth edition, page 405).

Another patent application no. WO2011076749A2 discloses a solid pharmaceutical composition of ticagrelor and asserts that ticagrelor particles having a particle size in the range of 1 pm to 150 pm exhibit beneficial effects in particular regarding an improved dissolution rate. However, it is stated that these effects are observable only if the ratio of ticagrelor is more that 50% by weight in the composition. The composition also comprises at least one hydrophilic polymer and an anti-thrombotic agent, in particular acetylsalicylic acid.

In the patent application no. WO2015001489A1, a pharmaceutical composition of ticagrelor is suggested to solve the problems regarding stability and dissolution which occur in case of the use of the amorphous form of ticagrelor.

Ticagrelor has a low aqueous solubility (10 µg/mL in water) and this situation brings bioavailability and stability problems regarding decreases in the dissolubility of the tablet formulations. In the state of the art, several methods are followed, different forms of ticagrelor and different excipients are used to compose innovative formulations and to overcome some of these problems. However, the challenge is to formulate a composition, some qualities of which won't reduce, while the others are improved. Thus, there is a need in the art for a pharmaceutical composition comprising ticagrelor providing enhanced dissolution profile, stability and bioavailability at the same time.

### Objects and Brief Description of the Invention

The main object of the present invention is to obtain ticagrelor formulations eliminating all aforesaid problems and bringing additional advantages to the relevant prior art.

Another object of the present invention is to obtain ticagrelor formulations with high stability and bioavailability.

A further object of the present invention is to develop solid oral formulations of ticagrelor having an improved level of dissolution rate, solubility and permeability.

A further object of the present invention is to develop solid oral formulations of ticagrelor ensuring the bioequivalence.

According to these objects, another object of the present invention is to obtain a solid oral pharmaceutical composition which is substantially free of calcium salts and their derivatives.

According to these objects, another object of the present invention is to obtain a solid oral pharmaceutical composition comprising ticagrelor and at least two binders.

Yet, another object of the present invention is to provide a ticagrelor tablet comprising at least one film coating to protect the pharmaceutical composition against the moisture to maintain the stability.

A further object of the present invention is to improve a process for preparing the said ticagrelor tablet, comprising wet granulation method which is performed by granulating the active agent in the hydrophilic polymers and which increases the solubility thereby provides the bioequivalence.

### Detailed Description of the Invention

In accordance with the objects outlined above, detailed features of the present invention are given herein.

The present invention relates to solid oral pharmaceutical compositions comprising ticagrelor or a pharmaceutically acceptable salt thereof as the active agent, wherein the composition is substantially free of calcium salts and their derivatives.

According to the preferred embodiment of the invention, the composition is further free of starch and its derivatives.

According to the preferred embodiment, the active agent is ticagrelor.

According to this preferred embodiment, the amount of ticagrelor is between 5-60% by weight of the total composition. Preferably this amount is between 10-50% by weight of the total composition. More preferably ticagrelor is present between 20-40% by weight in the total composition.

According to this embodiment, ticagrelor is present in an amount of between 1 to 300 mg, preferably 10 to 240 mg and more preferably it is 20 to 120 mg.

According to these embodiments, the composition is in the form of coated tablet, trilayer tablet, bilayer tablet, multilayer tablet, orally disintegrating tablet, mini tablet, pellet, sugar pellet, buccal tablet, sublingual tablet, effervescent tablet, immediate release tablet, modified release tablet, film-coated tablet, gastric disintegrating tablet, pill, capsule, oral granule, powder, coated bead system, microsphere, tablet in tablet, inlay tablet, dragee, sachet, orally administrable film.

The composition is preferably in the form of a film-coated tablet.

According to one embodiment, the composition further comprises at least one hydrophilic polymer.

According to this embodiment, the composition comprises at least one pharmaceutically acceptable excipient selected from binders, fillers, disintegrants, lubricants, glidants or mixtures thereof.

According to one embodiment, the solid oral pharmaceutical composition comprises at least two binders which are selected from the group comprising copovidone, copolyvidone, polyvinylpyrrolidone (PVP), povidone K30, carnauba wax, hydroxypropyl methyl cellulose (hypromellose, HPMC), pullulan, polymethacrylate, glyceryl behenate, hydroxypropyl cellulose (HPC), carboxymethyl cellulose (CMC), methyl cellulose (MC), hydroxyethyl cellulose, sodium carboxymethyl cellulose (Na CMC), ethyl cellulose, microcrystalline cellulose, polymetacrylates, polyethylene oxide, polyvinyl alcohol, polycarbophil, polyvinyl acetate and its copolymers, gelatin, xanthan gum, guar gum, alginate, carrageen, kollagen, agar, pectin, hyaluronic acid, carbomer, cellulose acetate phthalate, hydroxyethyl methyl cellulose, polaxomer, polyethylene glycol (PEG), sugars, glycose syrups, natural gums, tragacanth gum, polyacrylamide, aluminum hydroxide, benthonite, laponite, setostearyl alcohol, polyoxyethylene-alkyl ethers, acacia mucilage, polydextrose or mixtures thereof.

According to a preferred embodiment, the solid oral pharmaceutical composition comprises two binders which are microcrystalline cellulose and hydroxypropyl methylcellulose.

The amount of microcrystalline cellulose is between 5-50%, preferably 10-40%, more preferably 20-30% by weight of the total composition.

The amount of hydroxypropyl methylcellulose is between 1-20%, preferably 2-10% by weight of the total composition. Hydroxypropyl methylcellulose also takes part as a dissolution enhancer and it is selectively used as a binder for this purpose.

In the preferred embodiment, the ratio of ticagrelor to the total weight of binder is in the range of 2:1 to 0.5:1 and preferably 1.5:1 to 0.7:1 and more preferably 1.2:1 to 0.9:1.

According this preferred embodiment, the ratio of microcrystalline cellulose to the hydroxypropyl methylcellulose is in the range of 10:1 to 2:1 and preferably 8:1 to 4:1 and more preferably 7:1 to 5:1.

These preferred selections of range assure the enhanced stability and bioavailability during the shelf life.

According to one embodiment, the solid oral pharmaceutical composition comprises at least one filler which is selected from the group comprising lactose monohydrate, lactose, mannitol, spray-dried mannitol, dextrose, sucrose, fructose, maltose, sorbitol, xylitol, inositol, kaolin, inorganic salts, polysaccharides, sodium chloride, dextrates, lactitol, maltodextrin, sucrose-maltodextrin mixture, trehalose, sodium carbonate, sodium bicarbonate or mixtures thereof.

According to a preferred embodiment, the solid oral pharmaceutical composition comprises one filler which is mannitol.

The amount of mannitol is between 10-60%, preferably 15-50% and more preferably 30-40% by weight of the total composition.

According to one embodiment, the solid oral pharmaceutical composition comprises at least one disintegrant which is selected from the group comprising croscarmellose sodium, sodium carbonate, hydroxylpropyl cellulose (HPC), cross-linked polyvinylpyrrolidone (crospovidon), copovidon, polycarbophil, low-substitue poloxamer, sodium starch glycolate, alginic acid and alginates, ion-exchange resins, magnesium aluminum silica, sodium dodecyl sulphate, sodium carboxy methyl cellulose, docusate sodium, guar gum, polyacrylin potasium, sodium alginate, sodium glysin carbonate, sodium lauryl sulphate or mixtures thereof.

According to a preferred embodiment, the solid oral pharmaceutical composition comprises one disintegrant which is croscarmellose sodium.

The amount of croscarmellose sodium is between 0.5-15%, preferably 3-10% by weight of the total composition.

In the preferred embodiment, the ratio of croscarmellose sodium to ticagrelor is in the range of 1:2 to 1:10 and preferably 1:4 to 1:8 and more preferably 1:5 to 1:6.

According to this embodiment, the ratio of croscarmellose sodium to the total weight of binder is in the range of 1:2 to 1:10 and preferably 1:4 to 1:8 and more preferably 1:5 to 1:6.

These preferred selections of range are observed as increasing the solubility of ticagrelor and ensure the bioequivalence of the formulation accordingly.

According to one embodiment, the solid oral pharmaceutical composition comprises at least one lubricant and at least one glidant which are selected from the group comprising sodium lauryl sulphate, sodium stearyl fumarate, colloidal silicon dioxide, magnesium stearate, zinc stearate, mineral oil, talc, polyethylene glycol, glyceryl monostearate, glyceryl palmitostearate, magnesium lauryl sulphate, fumaric acid, zinc stearate, stearic acid, hydrogenated natural oils, silica, paraffin or mixtures thereof.

According to a preferred embodiment of the invention, the solid oral pharmaceutical composition comprises one lubricant which is sodium stearyl fumarate.

The amount of sodium stearyl fumarate is between 0.1-5%, preferably 1-3% by weight of the total composition.

According to a preferred embodiment of the invention, the solid oral pharmaceutical composition comprises one glidant which is colloidal silicon dioxide.

The amount of colloidal silicon dioxide is between 0.1-5%, preferably 1-3% by weight of the total composition.

According to these embodiments, the solid oral pharmaceutical composition comprises at least one coating layer to protect the composition against the moisture and maintain the stability. Suitable coating ingredients are selected from the group comprising hydroxypropyl methylcellulose (hypromellose), hydroxypropyl cellulose, polyvinyl alcohol (PVA), polyethylene glycol (PEG), talc, polyvinyl alcohol-polyethylene glycol copolymers (Kollicoat IR), ethylcellulose dispersions (Surelease), polyvinylprolidone, polyvinylprolidone-vinyl acetate copolymer (PVP-VA) and all kinds of OpadryTM, pigments, dyes, titanium dioxide, iron oxide or polymethylmetacrylate copolymers (Eudragit) and mixtures thereof.

According to a preferred embodiment, coating layer is Opadry Yellow which comprises hydroxypropyl methylcellulose, titanium dioxide, iron oxide yellow and polyethylene glycol.

According to the preferred embodiment, the solid oral pharmaceutical composition comprises ticagrelor as active agent, microcrystalline cellulose and hydroxypropyl methylcellulose as binders, mannitol as filler, croscarmellose sodium as disintegrant, sodium stearyl fumarate as lubricant, colloidal silicon dioxide as glidant and a coating layer.

According to this preferred embodiment, the composition comprises;
- 5-60% by weight ticagrelor,
- 5-50% by weight of microcrystalline cellulose,
- 1-20% by weight of hydroxypropyl methylcellulose,
- 10-60% by weight of mannitol,
- 0.5-15% by weight of croscarmellose sodium,
- 0.1-5% by weight of sodium stearyl fumarate,
- 0.1-5% by weight of colloidal silicon dioxide,
- 1-5% by weight of coating.

These analytically selected ratios ensure the required effective doses for the treatment and enhance the stability and dissolution profile of the film-coated tablet subjected to the invention.

According to all these embodiments, the below given formulations can be used in the solid oral pharmaceutical composition subjected to the invention.

### Example 1: Film-coated tablet formulation

| **Ingredients** | **Amount (%)** |
|---|---|
| Ticagrelor | 20 - 40 |
| Microcrystalline cellulose | 20 - 30 |
| Hydroxypropyl methylcellulose | 2 - 10 |
| Mannitol | 30 - 40 |
| Croscarmellose sodium | 3 - 10 |
| Sodium stearyl fumarate | 1 - 3 |
| Colloidal silicon dioxide | 1 - 3 |
| Coating | 1 - 5 |

| C**oating Material (Opadry yellow) Ingredients** | **Amount (%)** |
|---|---|
| Hydroxypropyl methylcellulose (6 cP) | 60 - 80 |
| Titanium dioxide | 25 - 35 |
| Iron oxide yellow / Ferroxide (510 P) | 3 - 10 |
| Polyethylene glycol powder (PEG 400) | 3 - 10 |

### Example 2: Film-coated tablet formulation

| **Ingredients** | **Amount (%)** |
|---|---|
| Ticagrelor | 29.0 |
| Microcrystalline cellulose | 24.0 |
| Hydroxypropyl methylcellulose | 4.0 |
| Mannitol | 34.0 |
| Croscarmellose sodium | 5.0 |
| Sodium stearyl fumarate | 2.0 |
| Colloidal silicon dioxide | 2.0 |

| **Total tablet** | **100** |
|---|---|
| Coating | 3.0 |
| **Coated tablet** | **103** |

| **Coating Material (Opadry yellow) Ingredients** | **Amount (%)** |
|---|---|
| Hydroxypropyl methylcellulose (6 cP) | 62.5 |
| Titanium dioxide | 24.95 |
| Iron oxide yellow / Ferroxide (510 P) | 6.30 |
| Polyethylene glycol powder (PEG 400) | 6.25 |

The preparation method of the above mentioned pharmaceutical formulations essentially comprises 4 main stages. During the first stage, basic granule structure which can also be called as internal phase is formed by wet granulation. These basic granules comprise the active ingredient and some of the excipients. In the second stage, these granules are processed with lubricant and glidant to form an external phase on them and ameliorate the final tablet product properties such as stability, wettability and disintegration. Third stage includes compressing these granules into tablets. In the fourth stage, a coating layer is performed on these tablets against the moisture to maintain the stability.

The pharmaceutical formulations subjected to the invention are prepared in detail by following these steps:
- mixing ticagrelor, mannitol, microcrystalline cellulose, hydroxypropyl methylcellulose, croscarmellose sodium together to prepare a powder mixture
- granulating the powder mixture with water
- drying the granules preferably until they have 1 to 3% moisture ratio
- sieving the dried granules through a sieving mesh
- adding sodium stearyl fumarate and colloidal silicon dioxide to the granulated mixture and mixing the total mixture
- compressing the total mixture into tablets
- preparing an aqueous solution of coating material and coating the tablets with this solution in a way to form a film layer

## Claims

1. A solid oral pharmaceutical composition comprising ticagrelor or a pharmaceutically acceptable salt thereof with at least one hydrophilic polymer, wherein the composition is substantially free of calcium salts and their derivatives.

2. The solid oral pharmaceutical composition according to claim 1, wherein the composition comprises ticagrelor in an amount of 5-60%, preferably 10-50% and more preferably 20-40% by weight.

3. The solid oral pharmaceutical composition according to claim 1 or 2, wherein the composition is in the form of coated tablet, trilayer tablet, bilayer tablet, multilayer tablet, orally disintegrating tablet, mini tablet, pellet, sugar pellet, buccal tablet, sublingual tablet, effervescent tablet, immediate release tablet, modified release tablet, film-coated tablet, gastric disintegrating tablet, pill, capsule, oral granule, powder, coated bead system, microsphere, tablet in tablet, inlay tablet, dragee, sachet, orally administrable film.

4. The solid oral pharmaceutical composition according to claim 3, wherein the composition is in the form of a film-coated tablet.

5. The solid oral pharmaceutical composition according to any preceding claims, wherein the composition further comprises at least one pharmaceutically acceptable excipient selected from binders, fillers, disintegrants, lubricants, glidants or mixtures thereof.

6. The solid oral pharmaceutical composition according to claim 5, wherein the composition comprises two binders which are microcrystalline cellulose and hydroxypropyl methylcellulose.

7. The solid oral pharmaceutical composition according to claim 6, wherein the ratio of ticagrelor to the total weight of binder is in the range of 2:1 to 0.5:1 and preferably 1.5:1 to 0.7:1 and more preferably 1.2:1 to 0.9:1.

8. The solid oral pharmaceutical composition according to claim 6, the ratio of microcrystalline cellulose to the hydroxypropyl methylcellulose is in the range of 10:1 to 2:1 and preferably 8:1 to 4:1 and more preferably 7:1 to 5:1.

9. The solid oral pharmaceutical composition according to claim 5, wherein the composition comprises one disintegrant which is croscarmellose sodium.

10. The solid oral pharmaceutical composition according to claim 9, the ratio of croscarmellose sodium to ticagrelor is in the range of 1:2 to 1:10 and preferably 1:4 to 1:8 and more preferably 1:5 to 1:6.

11. The solid oral pharmaceutical composition according to claim 9, the ratio of croscarmellose sodium to the total weight of binder is in the range of 1:2 to 1:10 and preferably 1:4 to 1:8 and more preferably 1:5 to 1:6.

12. The solid oral pharmaceutical composition according to any preceding claims, comprising;
- 5-60% by weight ticagrelor,
- 5-50% by weight of microcrystalline cellulose,
- 1-20% by weight of hydroxypropyl methylcellulose,
- 10-60% by weight of mannitol,
- 0.5-15% by weight of croscarmellose sodium,
- 0.1-5% by weight of sodium stearyl fumarate,
- 0.1-5% by weight of colloidal silicon dioxide,
- 1-5% by weight of coating.

13. A process for preparing the solid oral pharmaceutical composition according to claim 12, comprising the following steps:
- mixing ticagrelor, mannitol, microcrystalline cellulose, hydroxypropyl methylcellulose, croscarmellose sodium together to prepare a powder mixture
- granulating the powder mixture with water
- drying the granules
- sieving the dried granules through a sieving mesh
- adding sodium stearyl fumarate and colloidal silicon dioxide to the granulated mixture and mixing the total mixture
- compressing the total mixture into tablets
- preparing an aqueous solution of coating material and coating the tablets with this solution in a way to form a film layer
